# EUROPÄISCHE PATENTSCHRIFT

(11) **EP 2 414 821 B1**
(45) Veröffentlichungstag und Bekanntmachung des Hinweises auf die Patenterteilung: **14.08.2013**
(21) Anmeldenummer: 10711170.0
(22) Anmeldetag: 02.03.2010
(51) Int. Cl.: G01N 27/327

(54) **VORRICHTUNG NACH ART EINER ELEKTROCHEMISCHEN KAMERA SOWIE VERFAHREN ZUR HERSTELLUNG UND VERWENDUNG DER VORRICHTUNG**
DEVICE SIMILAR TO AN ELECTROCHEMICAL CAMERA AND METHOD FOR THE PRODUCTION AND USE OF THE DEVICE
DISPOSITIF DU TYPE CAMÉRA ÉLECTROCHIMIQUE, AINSI QUE PROCÉDÉ DE FABRICATION, ET UTILISATION DU DISPOSITIF

(30) Priorität: 31.03.2009 DE 102009015114
(43) Veröffentlichungstag der Anmeldung: 08.02.2012
(73) Patentinhaber: Siemens Aktiengesellschaft, 80333 München (DE)
(72) Erfinder: FREY, Alexander, 81737 München (DE); GUMBRECHT, Walter, 91074 Herzogenaurach (DE); PAULICKA, Peter, 91341 Röttenbach (DE); SCHIENLE, Meinrad, 85521 Ottobrunn (DE); STANZEL, Manfred, 91056 Erlangen (DE)
(86) Internationale Anmeldenummer: PCT/EP2010/052616
(87) Internationale Veröffentlichungsnummer: WO 2010/112287

(56) Entgegenhaltungen:
- WO-A1-2008/068678
- DE-A1- 10 259 821
- DE-A1-102004 019 357
- US-A1- 2008 132 429

## Beschreibung

Die vorliegende Erfindung bezieht sich auf eine Vorrichtung nach Art einer elektrochemischen Kamera zum Nachweis von chemischen oder biochemischen Substanzen in Flüssigkeiten sowie auf ein Verfahren zur Herstellung der Vorrichtung und deren Verwendung.

Der Nachweis von Substanzen in Flüssigkeiten gewinnt sowohl in der Chemie als auch in der Biochemie immer mehr an Bedeutung. Dabei werden vermehrt elektrochemische Sensoren eingesetzt, wie z.B. aus der DE 10 2004 019 357 A1 bekannt ist, welche aus Platzgründen auf einem Träger in Form eines Arrays angeordnet sind. Mögliche Materialien für die Träger sind Halbleitermaterialien, wie z. B. Silizium Si, Germanium Ge oder Gallium-Arsenid GaAs. Bei Einsatz dieser Materialien ermöglicht die Halbleitertechnik die Integration von Schaltungen zur Signalverarbeitung und Auswertung der Signale der elektrochemischen Sensoren auf demselben Träger, auf welchem sich das Sensor-Array befindet.

Da das Trägermaterial Si, Ge oder GaAs teuer und aufwendig herzustellen ist, wird versucht, Strukturen wie z. B. Sensoren so klein wie möglich auszubilden. Dabei ist darauf zu achten, dass eine zuverlässige Funktionsweise gewährleistet ist. In der Regel besteht ein Sensor aus fingerförmigen Interdigital-Elektroden, mit einer kleinsten Strukturbreite im Bereich von Mikrometern. Als Elektroden-Materialien kommen unter anderem Gold, Platin, Silber/Silber-Chlorid oder andere Metalle in Frage. Die Sensoren sind regelmäßig auf der Oberfläche des Trägers angeordnet, mit Abständen voneinander im Bereich von hundert Nanometern bis hin zu mehreren Millimetern.

Um chemische oder biochemische Substanzen in einer Flüssigkeit elektrochemisch nachweisen zu können, werden die Elektroden mit Molekülen beschichtet, welche mit den Substanzen wechselwirken. Die Wechselwirkung wird direkt oder indirekt elektrochemisch nachgewiesen. Unterschiedliche Sensoren sind mit verschiedenen Molekülen beschichtet, so dass verschiedene chemische oder biochemische Substanzen in der Flüssigkeit nachgewiesen werden können.

Die Beschichtung der Sensoren bzw. einzelner Elektroden erfolgt häufig durch Spotten von Molekülen auf die Sensoren. Mit geringer werdender Strukturgröße der Sensoren und Elektroden wird dies jedoch immer aufwendiger. Bei Elektrodengrößen im Nanometerbereich kann eine Beschichtung nicht mehr durch Spotten erfolgen. Teure und aufwendige Verfahren wie z. B. Photolithographie werden verwendet, um auf die Elektroden spezifisch Moleküle aufzubringen. Eine genaue Justierung der Einrichtungen bei der Beschichtung ist notwendig und macht das Herstellungsverfahren der Sensor-Arrays fehleranfällig und teuer.

Aufgabe der vorliegenden Erfindung ist es, eine Vorrichtung anzugeben, welche im Vergleich zum Stand der Technik einfacher und billiger herzustellen ist. Insbesondere ist es Aufgabe der Erfindung eine Vorrichtung anzugeben, bei welcher eine aufwendige Justierung bei In-Verbindung-Bringen der Moleküle zum Nachweis der chemischen oder biochemischen Substanzen mit den Sensoren entfällt. Weiterhin ist es Aufgabe der Erfindung, ein Verfahren zur Herstellung der Vorrichtung und eine Verwendung der Vorrichtung anzugeben, bei welchen ohne aufwendige Justierung, mit Hilfe von spezifisch bindenden Molekülen an die nachzuweisenden Substanzen, die Sensoren mit den Molekülen in direkte oder indirekte Verbindung z. B. über die Flüssigkeit gebracht werden können und beim Nachweis Sensoren spezifisch die Substanzen elektrochemisch nachweisen können.

Die angegebene Aufgabe wird bezüglich der Vorrichtung mit den Merkmalen des Anspruchs 1, bezüglich des Verfahrens zur Herstellung der Vorrichtung mit den Merkmalen des Anspruchs 15 und bezüglich der Verwendung der Vorrichtung mit den Merkmalen des Anspruchs 17 gelöst.

Vorteilhafte Ausgestaltungen der erfindungsgemäßen Vorrichtung, des erfindungsgemäßen Verfahrens und der erfindungsgemäßen Verwendung gehen aus den jeweils zugeordneten abhängigen Unteransprüchen hervor. Dabei können die Merkmale der nebengeordneten Ansprüche mit Merkmalen eines jeweils zugeordneten Unteranspruchs oder vorzugsweise auch mit Merkmalen mehrerer zugeordneter Unteransprüche kombiniert werden.

Die erfindungsgemäße Vorrichtung zum Nachweis von chemischen oder biochemischen Substanzen in Flüssigkeiten weist einen ersten und einen zweiten Träger auf. Eine Oberfläche des ersten Trägers umfasst ein Sensor-Array mit mehreren elektrochemischen Sensoren. Der zweite Träger weist eine poröse Schicht auf mit wenigstens einem funktionellen Bereich, in dem spezifisch bindende Fänger-Moleküle immobilisiert sind. Der wenigstens eine funktionelle Bereich ist direkt benachbart zu wenigstens einem nicht funktionalisierten Bereich angeordnet. Die Oberfläche des ersten Trägers und die poröse Schicht des zweiten Trägers stehen direkt oder indirekt über ein flüssiges Medium miteinander in Kontakt. Dem wenigstens einen funktionellen Bereich und dem wenigstens einen nicht funktionalisierten Bereich sind jeweils mehrere Sensoren des Sensor-Arrays zugeordnet.

Durch die Anordnung der Sensoren in Array-Form auf dem ersten Träger und durch die Zuordnung von jeweils mehreren Sensoren zu wenigstens einen funktionellen Bereich und zu wenigstens einen nicht funktionalisierten Bereich des zweiten Trägers kann bei einer elektrochemischen Messung räumlich aufgelöst zwischen unterschiedlichen Bereichen unterschieden werden. Eine genaue Justierung des ersten zum zweiten Träger beim In-Kontakt-Bringen kann entfallen.

Bei Einbringen der Flüssigkeit mit nachzuweisenden Substanzen in den zweiten Träger und einer elektrochemischen Messung mit

Hilfe der Sensoren des Sensor-Arrays ergeben die Sensoren, welche in Kontakt zu einem funktionellen Bereich liegen, ein erhöhtes Messsignal. Dieses ist durch die Wechselwirkung der nachzuweisenden Substanzen mit den Fänger-Molekülen bedingt. Sensoren, welche in Kontakt zu nicht funktionalisierten Bereichen stehen, ergeben kein Signal oder nur ein kleines, für die Hintergrundreaktionen typisches Signal.

Je mehr Sensoren den jeweiligen Bereichen zugeordnet sind, desto genauer kann die räumliche Auflösung der Bereiche und Zuordnung der chemischen oder biochemischen Reaktionen zu Bereichen erfolgen. Die Auflösung ist analog der Auflösung einer optischen Kamera. Mit einer höheren optisch aktiven Pixelzahl einer optischen Kamera wird eine höhere Auflösung eines aufgenommenen optischen Bildes erreicht. Eine genaue Justierung der Kamera auf das aufzunehmende Objekt ist nicht nötig. Die Helligkeits- bzw. Farbwerte der Pixel des aufgenommenen Bildes ergeben in der Gesamtbetrachtung Umrisse des Objektes, mit welchen dieses innerhalb des Bildes eindeutig identifiziert werden kann. Nach Identifizierung des Objektes, d.h. Unterscheidung vom Hintergrund, können dessen Eigenschaften genauer ausgewertet werden.

Analog der optischen Kamera funktioniert die erfindungsgemäße Vorrichtung, welche als elektrochemische Kamera bezeichnet werden kann. Jeweils einem Bereich sind mehrere Sensoren zugeordnet. Chemische oder biochemische Reaktionen in dem jeweiligen Bereich werden durch mehrere Sensoren gemessen und abgebildet. Die stark unterschiedlichen Reaktionen in einem funktionellen Bereich im Vergleich zu einem nicht funktionalisierten Bereich führen zu Signalen an zu dem funktionellen Bereich zugeordneten Sensoren, welche unterschiedlich sind zu Signalen, gemessen an Sensoren, zugeordnet zu einem nicht funktionalisierten Bereich. Durch die Anordnung der Sensoren in Array-Form und der damit verbundenen Kenntnis ihrer räumlichen Lage auf dem ersten Träger kann durch die Zuordnung der Sensoren zu den Bereichen zwischen der Lage unterschiedlicher Bereiche unterschieden werden. Über die räumliche Anordnung der Sensoren auf dem ersten Träger kann die räumliche Lage der Bereiche in der porösen Schicht des zweiten Trägers ermittelt werden.

Besonders bevorzugt weist die poröse Schicht des zweiten Trägers der erfindungsgemäßen Vorrichtung wenigstens zwei unterschiedliche, voneinander räumlich beabstandete funktionelle Bereiche auf. Dabei kann sich mindestens ein Bereich von mindestens einem weiteren Bereich hinsichtlich seiner Fänger-Moleküle unterscheiden. Dadurch wird der Nachweis unterschiedlicher chemischer oder biochemischer Substanzen möglich.

Ein besonders einfacher Aufbau der Vorrichtung und eine gute Zuordnung der Sensoren zu den Bereichen ergibt sich, wenn jeder Sensor des Sensor-Arrays eine aktive Fläche aufweist, welche in einer ersten Ebene angeordnet ist, und Oberflächen der wenigstens zwei funktionellen Bereiche eine zweite Ebene ausbilden, welche der ersten Ebene gegenüberliegend angeordnet ist. Dabei kann jeweils die Oberfläche eines funktionellen Bereichs in der zweiten Ebene größer als die Summe der aktiven Flächen von Sensoren sein, die jeweils dem funktionellen Bereich in der ersten Ebene zugeordnet sind. Auch ohne präzise Justierung ist durch diesen Aufbau der Vorrichtung bei In-Kontakt-Bringen des ersten mit dem zweiten Träger automatisch die Zuordnung mehrerer Sensoren zu jeweils einem Bereich gegeben.

Die Fänger-Moleküle können im porösen Material räumlich dreidimensional verteilt immobilisiert sein. Dadurch werden besonders viele Fänger-Moleküle auf engstem Raum im porösen Material angeordnet und die vielen Fänger-Moleküle können viel nachzuweisende Substanz binden. Dadurch wird das Signal-Rauschverhältnis bei der elektrochemischen Messung groß und man erhält einen großen Unterschied zwischen Signalen von funktionellen Bereichen und nicht funktionalisierten Bereichen.

Entsprechend der Idee einer elektrochemischen Kamera können die Sensoren in Form eines Pixel-Arrays regelmäßig auf der Oberfläche des ersten Trägers angeordnet sein. Dadurch wird die räumliche Zuordnung von Messsignalen einfacher auswertbar.

Besonders viel aktive Fläche der Sensoren lässt sich auf einer Oberfläche bestimmter Fläche anordnen, wenn die einzelnen Sensoren einen rechteckigen, besonders bevorzugt quadratischen Umfang aufweisen. Durch die relativ große aktive Fläche der Sensoren erhält man ein gutes Signal/Rauschverhältnis und relativ große Messsignale.

Eine einfache räumliche Zuordnung der Sensoren zu den Bereichen erhält man, wenn beabstandete funktionelle Bereiche in Form eines Arrays in und/oder auf der porösen Schicht angeordnet sind, mit einer Anzahl m von unterschiedlichen funktionellen Bereichen mit im Wesentlichen gleichem räumlichen Abstand jeweils voneinander. Bevorzugt weist das Sensor-Array eine Anzahl von n Sensoren auf, welche funktionellen Bereichen zugeordnet sind, und die Anzahl n der zugeordneten Sensoren zu funktionellen Bereichen beträgt das x-fache der Anzahl m der funktionellen Bereiche, mit x größer oder gleich zwei. Durch die regelmäßige Anordnung der funktionellen Bereiche und die Zuordnung der Sensoren des Sensor-Arrays wird die Auswertung der Messergebnisse und Interpretation des sich aus den Messergebnissen ergebenen Bildes erleichtert. Dadurch kann mit geringem Aufwand aus den Signalen der Sensoren und der Kenntnis ihrer Lage sowie der Kenntnis des Abstands der funktionellen Bereiche zueinander ein räumlich aufgelöstes Bild der chemischen oder biochemischen Reaktionen in dem zweiten Träger erhalten werden und den Messsignalen und/oder Bereichen Reaktionen zugeordnet werden.

Günstige Ausführungsformen der Vorrichtung, welche sich leicht herstellen lassen, sind gegeben, wenn eine Anzahl m der funktionellen Bereiche 8 beträgt und die Anzahl n der Sensoren 32, und jedem funktionellen Bereich 4 Sensoren zugeordnet sind, oder wenn die Anzahl m der funktionellen Bereiche 24 beträgt und die Anzahl n der Sensoren 384, und jedem funktionellen Bereich 16 Sensoren zugeordnet sind, oder wenn die Anzahl m der funktionellen Bereiche zwischen 8 und 24 beträgt und die Anzahl n der Sensoren zwischen 32 und 384 beträgt.

Die Sensoren können aus Interdigital-Elektroden aufgebaut sein. Als Elektrodenmaterial wird bevorzugt unbeschichtetes Gold verwendet. Bei diesem Aufbau lassen sich viele chemische und biochemische Reaktionsprodukte elektrochemisch mit großem Signal aus Strom oder Spannung messen. Gold als Elektrodenmaterial ist dabei auch über lange Zeit stabil.

Die poröse Schicht hat bevorzugt die Form eines Blattes (planare Form), insbesondere die Form eines Papier-Streifens. Die poröse Schicht kann z. B. 100 Mikrometer dick ausgebildet sein und eine Fläche von wenigen Quadratmillimetern aufweisen. Die funktionellen Bereiche können in Form von Streifen oder Balken ausgebildet sein, insbesondere quaderförmigen Balken, die begrenzt werden von zwei im wesentlichen parallelen, voneinander beabstandeten Schnittflächen entlang von Querschnittsachsen der porösen Schicht durch die poröse Schicht. Dabei sind die Streifen oder Balken der funktionellen Bereich bevorzugt senkrecht zu einer Längsachse der porösen Schicht, insbesondere des Papier-Streifens, angeordnet. Dadurch ergibt sich ein Aufbau des zweiten Trägers analog dem eines Teststreifens, wie er z. B. in Glukose- oder Schwangerschaftstests eingesetzt wird.

Die poröse Schicht ist vorzugsweise aus einer Membran aufgebaut oder umfasst eine Membran, insbesondere aus Zellulose, Nitro-Zellulose, Lateral-Flow-Papier oder einem Gewebe. Diese Materialien sind in der Lage Fänger-Moleküle zu binden sowie Flüssigkeiten durch Kapillarkräfte aufzusaugen und durch das Material zu transportieren. Dadurch werden für die Funktionsweise der erfindungsgemäßen Vorrichtung keine Einrichtungen zum Transport der Flüssigkeit, wie z. B. Pumpen, benötigt. Natürlich kann der Einsatz der zuvor erwähnten Einrichtungen zusätzlich erfolgen, um einen schnelleren oder gleichmäßigeren Flüssigkeitstransport zu erreichen.

Als Fänger-Moleküle können unter anderem Antikörper oder Antigene, Aptamere, DNA-Fragmente, RNA-Fragmente, oder Peptide bzw. Kombinationen dieser Moleküle verwendet werden. Die Auswahl der Fänger-Moleküle ist abhängig von den mit der Vorrichtung nachzuweisenden chemischen oder biochemischen Substanzen.

Bei dem erfindungsgemäßen Verfahren zum Herstellen der Vorrichtung mit einem ersten Träger, welcher ein Sensor-Array umfasst, das aus mehreren elektrochemischen Sensoren aufgebaut ist, wird ein zweiter Träger aufgebracht. Der zweite Träger umfasst wenigstens einen funktionellen Bereich in oder auf einer porösen Schicht, in dem spezifisch bindende Fänger-Moleküle immobilisiert sind. Der wenigstens eine funktionelle Bereich wird direkt benachbart zu wenigstens einem nicht funktionalisierten Bereich in oder auf der porösen Schicht angeordnet. Durch das Aufbringen werden dem wenigstens einen funktionellen Bereich und dem wenigstens einen nicht funktionalisierten Bereich jeweils mehrere Sensoren des Sensor-Arrays zugeordnet.

Vorteilhaft ist dabei, dass der erste Träger mit dem Sensor-Array wiederverwendet werden kann. Der zweite Träger kann als Einwegartikel verwendet werden und nach jeder Benutzung der Vorrichtung ausgewechselt werden. Es können mit ein und demselben ersten Träger verschiedene Tests mit unterschiedlich funktionalisierten zweiten Trägern durchgeführt werden. Es können auch mit ein und demselben ersten Träger Tests mit z. B. Proben von unterschiedlichen Patienten durchgeführt werden. Dabei werden die zweiten Träger jeweils nach einer Untersuchung einer Patientenprobe ausgewechselt. Falls nötig kann der erste Träger zwischen zwei Untersuchungen gereinigt und desinfiziert werden, da insbesondere Gold und Siliziumoxid Oberflächen des ersten Trägers und des Sensor-Arrays sehr unempfindlich sind gegenüber Reinigungsmitteln und Desinfektionsmitteln sowie gegenüber DNA, RNA und Protein abbauenden Mitteln. Sie werden durch diese im Gegensatz zu organischen Beschichtungen nicht zerstört. Die organischen Fänger-Moleküle, welche im Stand der Technik als Beschichtung auf den Sensoren direkt aufgebracht sind, sind bei dem erfindungsgemäßen Verfahren in dem porösen zweiten Träger enthalten, und werden bei einer Reinigung des ersten Trägers nicht beschädigt.

Besonders vorteilhaft ist das erfindungsgemäße Verfahren bei Ausführung des Verfahrens in zeitlich aufeinanderfolgenden Schritten. Der wenigstens eine funktionelle Bereich wird in einem ersten Schritt in die poröse Schicht durch Dispensieren, Drucken und/oder Spotten eingebracht. In einem zweiten, zeitlich darauffolgenden Schritt wird die poröse Schicht in direkte oder indirekte Verbindung gebracht mit dem Sensor-Array des ersten Trägers. Dies kann insbesondere durch Auflegen des zweiten Trägers auf den ersten Träger erfolgen. Bei Einbringen von Flüssigkeit in die poröse Schicht tritt der wenigstens eine funktionelle Bereich und der wenigstens eine nicht funktionalisierte Bereich jeweils mit den mehreren zugeordneten Sensoren über die Flüssigkeit miteinander in Verbindung. In Verbindung treten bedeutet in diesem Fall, dass Moleküle wie z. B. Reaktionsprodukte von chemischen oder biochemischen Reaktionen, welche das Binden der zu untersuchenden Substanzen an die Fänger-Moleküle anzeigen, zu den elektrochemischen Sensoren (z. B. durch Diffusion oder elektrische Felder bei geladenen Molekülen) gelangen können. An den elektrochemischen Sensoren werden dann die Moleküle z. B. der Reaktionsprodukte elektrochemisch nachgewiesen. Dadurch erfolgt direkt oder bei Reaktionsprodukten indirekt ein Nachweis der chemischen oder biochemischen Substanzen in der zu untersuchenden Flüssigkeit in dem Fall, dass die Substanzen von den Fängermolekülen gebunden wurden.

Bei der erfindungsgemäßen Verwendung der Vorrichtung wird eine Flüssigkeit mit nachzuweisenden Substanzen auf einen zweiten Träger beaufschlagt, welcher eine poröse Schicht mit wenigstens einem funktionellen Bereich umfasst, in dem spezifisch bindende Fänger-Moleküle zum Binden an die nachzuweisenden Substanzen immobilisiert sind. Der wenigstens eine funktionelle Bereich ist direkt benachbart zu wenigstens einem nicht funktionalisierten Bereich in der porösen Schicht angeordnet. Die Flüssigkeit wird insbesondere durch Kapillarkräfte zumindest teilweise durch die poröse Schicht hindurchtransportiert, wobei die nachzuweisenden Substanzen spezifisch an die Fänger-Moleküle binden. Mit Hilfe von Sensoren eines Sensor-Arrays auf einem zweiten Träger, welche dem wenigstens einem funktionellen Bereich zugeordneten sind, werden die gebundenen Substanzen direkt oder indirekt elektrochemisch nachgewiesen. Dabei weisen die Sensoren des Sensor-Arrays auf dem zweiten Träger, welche dem wenigstens einem nicht funktionalisierten Bereich zugeordnet sind, elektrochemisch nach, dass keine nachzuweisenden Substanzen in dem nicht funktionalisierten Bereich gebunden wurden.

Für das erfindungsgemäße Verfahren zum Herstellen der Vorrichtung und die Verwendung der Vorrichtung ergeben sich die vorstehend erwähnten, mit der erfindungsgemäßen Vorrichtung verbundenen Vorteile.

Bevorzugte Ausführungsformen der Erfindung mit vorteilhaften Weiterbildungen gemäß den Merkmalen der abhängigen Ansprüche werden nachfolgend anhand der folgenden Figuren näher erläutert, ohne jedoch darauf beschränkt zu sein.

Es zeigen:
- Fig. 1: schematisch eine Schrägansicht einer Explosionszeichnung der erfindungsgemäßen Vorrichtung mit einem ersten und einem zweiten Träger, und
- Fig. 2: schematisch eine Aufsicht auf den zweiten Träger mit kreisrunden funktionellen Bereichen, wobei der sich darunter befindende erste Träger mit Sensor-Array als Durchsicht durch den zweiten Träger abgebildet ist, und
- Fig. 3: schematisch eine Aufsicht auf den zweiten Träger analog Fig. 2, jedoch mit streifen- oder balkenförmigen funktionellen Bereichen, und
- Fig. 4: schematisch eine Schnittdarstellung durch die Vorrichtung entsprechend Fig. 3, und
- Fig. 5: schematisch eine Schnittdarstellung der Vorrichtung kurz bevor der zweite Träger in Verbindung zum ersten Träger gebracht wird, und
- Fig. 6: eine Schnittdarstellung der Vorrichtung mit Vergrößerter Abbildung eines Sensors mit Interdigital-Elektroden sowie eine schematische Darstellung der Nachweisreaktion in einem funktionellen Bereich.

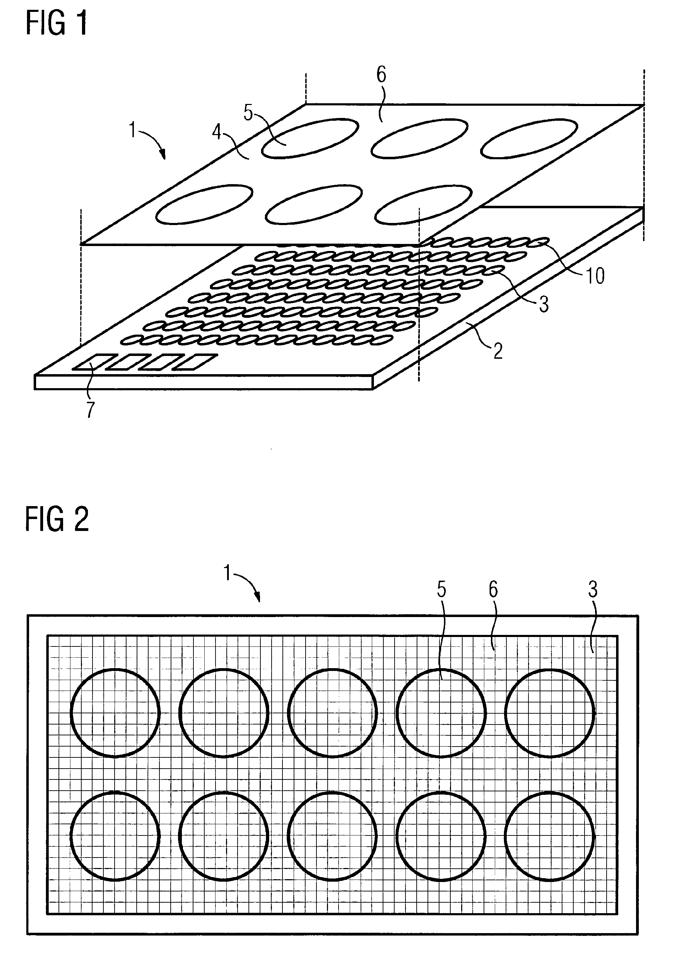

Fig. 1 zeigt die erfindungsgemäße Vorrichtung 1. Die Vorrichtung 1 weist einen ersten Träger 2 auf, auf dessen Oberfläche ein Sensor-Array 3 mit elektrochemischen Sensoren 10 ausgebildet ist. Die elektrochemischen Sensoren 10 sind kreisförmig ausgebildet und regelmäßig, in gleichen Abständen voneinander auf der Oberfläche des ersten Trägers 2 angeordnet.

Auf bzw. in dem ersten Träger 2 sind elektrische Verbindungen, welche der Einfachheit halber nicht dargestellt sind, zu elektrischen Anschlüssen 7 ausgebildet. Die elektrischen Anschlüsse 7 ermöglichen einen elektrischen Anschluss des ersten Trägers 2 an eine nicht dargestellte Spannungsversorgung sowie Steuer-/Auslese- und/oder Datenverarbeitungs-Einheit. Die elektrochemischen Sensoren 10 können über die Anschlüsse 7 und die elektrischen Verbindungen einzeln oder parallel angesteuert und ausgelesen werden. Eine Verarbeitung von Messsignalen, wie z. B. Strom-Spannungs-Werten der Sensoren 10, kann extern in der Auslese- und Datenverarbeitungs-Einheit erfolgen. Alternativ können auch integrierte Schaltungen auf oder in dem ersten Träger ausgebildet sein, welche elektrochemische Signale verarbeiten und die Ergebnisse als elektrische Signale über die Kontakte 7 an eine externe, nicht dargestellte Anzeige-Einheit ausgeben.

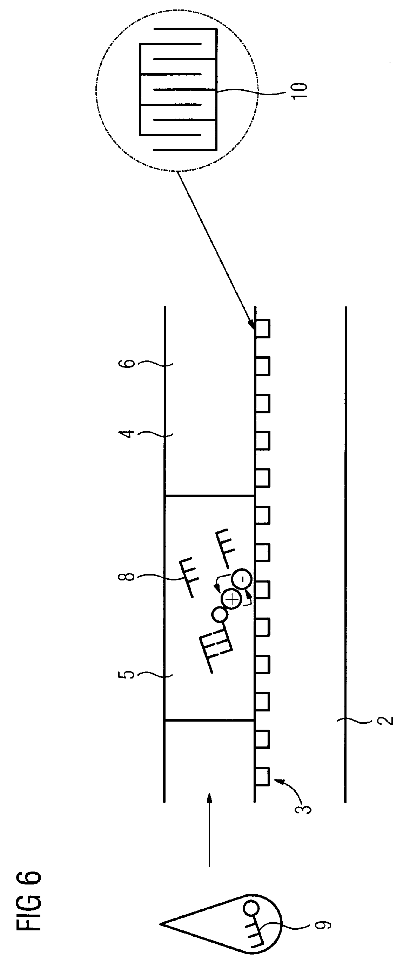

Oberhalb der Oberfläche des ersten Trägers 2 mit Sensor-Array 3 befindet sich gemäß Fig. 1 eine poröse Schicht 4 eines zweiten Trägers. In der porösen Schicht 4 sind in gleichen Abständen voneinander, in Array-Form funktionelle Bereiche 5 ausgebildet, in welchen Fänger-Moleküle 8 (vgl. Fig. 6) immobilisiert sind. Die funktionellen Bereiche 5 sind durch einen nicht funktionalisierten Bereich 6 voneinander getrennt. Die in Fig. 1 dargestellten funktionellen Bereiche 5 sind kreisförmig ausgebildet, in einer flachen, blattförmigen Schicht 4. Blattförmig bedeutet in diesem Zusammenhang, dass die Form analog der eines Blattes Papier ist. Die Ausdehnung des Materials der porösen Schicht 4 ist in einer Ebene erheblich größer als in der Senkrechten zu der Ebene. Die in Fig. 1 dargestellten kreisförmigen funktionellen Bereiche 5 stellen somit zylinderförmige Bereiche dar, welche aber eine zu ihrer Grundfläche fast vernachlässigbare Mantelfläche aufweisen.

Die Grundflächen der funktionellen Bereiche 5, welche gegenüberliegend zu der Ebene der Sensoren 10 angeordnet sind, liegen in einer Ebene. Diese Ebene, im Weiteren die Ebene der funktionellen Bereiche genannt, ist parallel zu der Ebene der Sensoren 10 des Sensor-Arrays. Die Oberfläche (bzw. Grundfläche) eines funktionellen Bereiches 5 in der Ebene der funktionellen Bereiche ist größer der aktiven Fläche eines Sensors 10. Mit aktiver Fläche eines Sensors 10 ist dabei die Fläche bezeichnet, welche bei der elektrochemischen Messung mit Flüssigkeit in Kontakt steht und somit an der Messung aktiv beteiligt ist, indem chemische Reaktionen an ihr bzw. Ladungsdurchtritt durch diese stattfinden kann.

Im Gebrauch der Vorrichtung 1 liegt die poröse Schicht 4 des zweiten Trägers direkt auf dem ersten Träger 2 und somit auf dem Sensor-Array 3. Die Kontakte 7 liegen beabstandet zu der porösen Schicht 4, damit sie bei einer Verwendung der Vorrichtung nicht in Kontakt mit Flüssigkeit gelangen und keinen elektrischen Kurzschluss verursachen. Sie können auch auf der Rückseite des ersten Trägers 2 angeordnet sein. Die gestrichelten Linien in Fig. 1 geben die räumliche Zuordnung der porösen Schicht des zweiten Trägers 4 relativ zum ersten Träger 2 wieder, wenn der zweite Träger 4 auf den ersten Träger 2 aufgelegt wird.

In Fig. 2 ist schematisch eine Aufsicht auf die in Fig. 1 abgebildete erfindungsgemäße Vorrichtung 1 dargestellt. Dabei sind die Kontakte 7 der Einfachheit halber nicht gezeigt. Es ist eine Situation wiedergegeben, bei welcher die poröse Schicht 4 des zweiten Trägers in direktem Kontakt mit dem ersten Träger 2 steht. Der zweite Träger 4, mit kreisrunden funktionellen Bereichen 5, ist transparent dargestellt, damit der sich darunter befindende erste Träger 2 mit Sensor-Array 3 als Durchsicht durch den zweiten Träger 4 darstellen lässt. In der Regel ist der zweite Träger 4 nicht optisch transparent ausgebildet.

Die in Fig. 2 dargestellten Sensoren 10 sind quadratisch, mit geringem Abstand voneinander angeordnet, wobei sie ein Pixel-Array ausbilden, analog dem eines optischen Kamera-Chips zur Aufnahme optischer Bilder. Das Sensor-Array 3 steht in Kontakt mit der porösen Schicht 4 des zweiten Trägers, wobei im Grundzustand die Poren der porösen Schicht mit Luft gefüllt sind. Bei Befüllen der Vorrichtung mit der zu untersuchenden Flüssigkeit wird z. B. durch Kapillarkräfte die Flüssigkeit in die Poren, welche zusammenhängend sind, gezogen. Nach vollständigem Befüllen stehen die Sensoren 10 des Sensor-Arrays 3 direkt, oder indirekt über die Flüssigkeit, in Kontakt mit der porösen Schicht 4 des zweiten Trägers.

Die funktionellen Bereiche 5 in der Fig. 2 sind in der Aufsicht kreisrund ausgebildet und in gleichem Reihen-Abstand voneinander, in Array-Form in der porösen Schicht 4 angeordnet. In den funktionellen Bereichen 5 sind Fänger-Moleküle immobilisiert, d.h. gleichmäßig verteilt an das poröse Material gebunden. Mögliche Bindungen können z. B. durch Coulomb-Wechselwirkungen, kovalente Bindungen oder durch die Einschränkung der Beweglichkeit von großen Molekülen in kleinen Poren erfolgen. Die funktionellen Bereiche 5 sind durch einen nicht funktionalisierten Bereich 6, welcher zusammenhängend ausgebildet ist, in der porösen Schicht 4 entlang der Ebene der funktionellen Bereiche betrachtet, vollständig umschlossen. Senkrecht zur Ebene der funktionellen Bereiche sind die funktionellen Bereich 5 vollständig durch die poröse Schicht 4 durchgehend ausgebildet.

Beim Auflegen des zweiten Trägers 4 auf den ersten Träger 2 werden den funktionellen Bereichen 5 Sensoren 10 zugeordnet. Zugeordnet bedeutet in der Aufsicht der Fig. 2, sie liegen innerhalb der kreisrunden funktionellen Bereiche 5. Analog werden beim Auflegen des zweiten Trägers 4 auf den ersten Träger 2 dem nicht funktionalisierten Bereich 6 Sensoren 10 zugeordnet. Sie liegen innerhalb des nicht funktionalisierten Bereichs 6 in Fig. 2, und außerhalb der kreisrunden funktionellen Bereiche 5.

Wird die Vorrichtung in Fig. 2 mit Flüssigkeit befüllt, welche nachzuweisende Substanzen 9 enthält, so binden diese an Fänger-Moleküle 8 (vgl. Fig. 6) spezifisch in den funktionellen Bereichen 5, in denen die spezifisch bindenden Fänger-Moleküle 8 für diese Substanzen 9 immobilisiert sind. Bei einer elektrochemischen Messung ergeben Sensoren 10, welche diesen Bereichen zugeordnet sind Signale, welche die spezifische Bindung der nachzuweisenden Substanze 9 anzeigen. Sensoren 10, welche dem nicht funktionalisierten Bereich 6 zugeordnet sind, messen nur Hintergrundreaktionen, wie z. B. die Ausbildung und Umladung von Doppelschichten. Funktionelle Bereiche 5, in denen keine nachzuweisenden Substanzen 9 gebunden haben, messen ebenfalls nur Hintergrundreaktionen. Fehler, wie sie z. B. entstehen durch Diffusion und Bewegungen im elektrischen Feld von Reaktionsprodukten beim Nachweis der spezifisch gebundenen Substanzen 9, seinen in dieser Betrachtung vernachlässigt.

Messen die zu einem funktionellen Bereich 5 zugeordneten Sensoren 10 große Signale, z. B. bei amperometrischen Messungen große Ströme, direkt oder indirekt durch die Bindung einer nachzuweisenden Substanz 9 an die spezifischen Fängermoleküle 8, welche in dem funktionellen Bereich 5 immobilisiert sind, so können Sensoren 10 im nicht funktionalisierten Bereich 6 kleine Signale, z. B. kleine Ströme messen. Sensoren 10, welche auf dem Umfang des kreisförmigen funktionellen Bereichs 5 liegen, messen Signale bzw. Ströme, welche zwischen den Signalen bzw. Strömen der Sensoren 10 der funktionellen Bereiche 5 und Signalen bzw. Strömen der Sensoren 10 der nicht funktionalisierten Bereiche 6 liegen. Dabei kann die Signal- bzw. Stromgröße ein Maß für die gemeinsame Fläche zwischen Sensor 10 und funktionellem Bereich 5 sein.

Eine präzise Justierung des zweiten Trägers 4 beim Aufbringen auf bzw. in Kontaktbringen mit dem ersten Träger 2 kann entfallen. Aufgrund der Anordnung der Sensoren 10 in Array-Form und einer Zuordnung von mehr als einem Sensor 10 jeweils zu einem funktionellen Bereich 5 und einem nicht funktionalisierten Bereich 6, ist bei einer elektrochemischen Messung anhand der Signale die Lage der Bereiche relativ zu den Sensoren zu unterscheiden. Zusätzlich eingebrachte Substanzen in den funktionalisierten Bereichen 5, welche elektrochemisch aktiv sind, jedoch den elektrochemischen Nachweis der Bindung der nachzuweisenden Substanzen 9 in der Flüssigkeit nicht behindern bzw. verhindern, können eine Unterscheidung weiter verbessern. So können auch funktionelle Bereiche 5 vom nicht funktionalisierten Bereich 6 unterschieden werden, in welchen keine Substanzen aus der Flüssigkeit binden, da diese nachzuweisenden Substanzen nicht in der Flüssigkeit enthalten sind (negativ Test).

Die Sensoren 10 sind in Array-Form angeordnet mit regelmäßigen Abständen voneinander, wobei die Abstände so gering wie möglich ausgebildet sein sollten, jedoch einen Wert aufweisen, welcher ein Übersprechen als Problem bei der elektrochemischen Messung minimiert. Je mehr Sensoren 10 auf der Oberfläche mit geringem Abstand angeordnet sind, desto höher wird die räumliche elektrochemische Auflösung (analog der optischen Auflösung bei Kameras) und Unterscheidung der funktionellen Bereiche 5. Typische Werte für den Abstand der Sensoren liegen im Bereich von Mikrometern, insbesondere 100 bis 1000 Mikrometern. Für eine höhere Auflösung können die Abstände auch im Bereich von Nanometern ausgebildet werden. Bei Untersuchungen, welche keine hohe räumliche Auflösung benötigen, sind auch Abstände im Millimeter bis hin zum ZentimeterBereich möglich.

Durch die Anordnung der Sensoren 10 in Array-Form mit geringem Abstand und funktionellen Bereichen 5 sowie nicht funktionalisierten Bereichen 6, welche wesentlich größer sind als die aktive Fläche eines Sensors 10, ist bei Aufbringen des zweiten Trägers 4 auf den ersten Träger 2 sichergestellt, dass jedem funktionellen Bereich 5 sowie jedem nicht funktionalisierten Bereich 6 mehrere Sensoren 10 zugeordnet sind, d.h. dass in der Aufsicht in Fig. 2 jeweils mehrer Sensoren 10 unterhalb eines jeweiligen Bereiches 5, 6 liegen. So ist unabhängig von der genauen Lage des ersten Trägers 2 relativ zum zweiten Träger 4 sichergestellt, dass bei einer elektrochemischen Messung für jeden Bereich 5, 6 Sensoren 10 ein Messsignal liefern. Die Größe des Messsignals ergibt in der Auswertung, welcher Sensor 10 welchem Bereich 5, 6 zugeordnet ist. In der Gesamtheit der Sensoren 10 ergibt sich bei Kenntnis ihrer räumlichen Anordnung ein elektrochemisches Bild des zweiten Trägers 4, analog einem optischen Bild bei Abbildung von Objekten mit Hilfe einer optischen CCD-Kamera.

Anhand der sich ergebenden Umrisse bzw. der Lage der Sensoren 10, zwischen welchen sich stark unterscheidende Signale gemessen werden, können Bereiche 5, 6 voneinander unterschieden werden bzw. voneinander abgegrenzt und räumlich zugeordnet werden, und an Hand der Größe der Signale können Bereiche 5, 6 identifiziert werden. Die Größe des Signals ist analog dem Grauwert in einem optischen Schwarz/Weiß-Bild. Objekte können von ihrem Hintergrund unterschieden werden durch große Unterschiede benachbarter Werte, welche Umrisse wiedergeben, und identifiziert werden an Hand der Struktur bzw. Verteilung der Werte innerhalb eines Umrisses (ergibt z. B. ein Gesicht oder ein Haus ... in einem Bild) und der Form des Umrisses. Analoges gilt für die elektrochemischen Messwerte der Sensoren 10 des Sensor-Arrays 3, welche ein elektrochemisches Abbild des zweiten Trägers 4 ergeben.

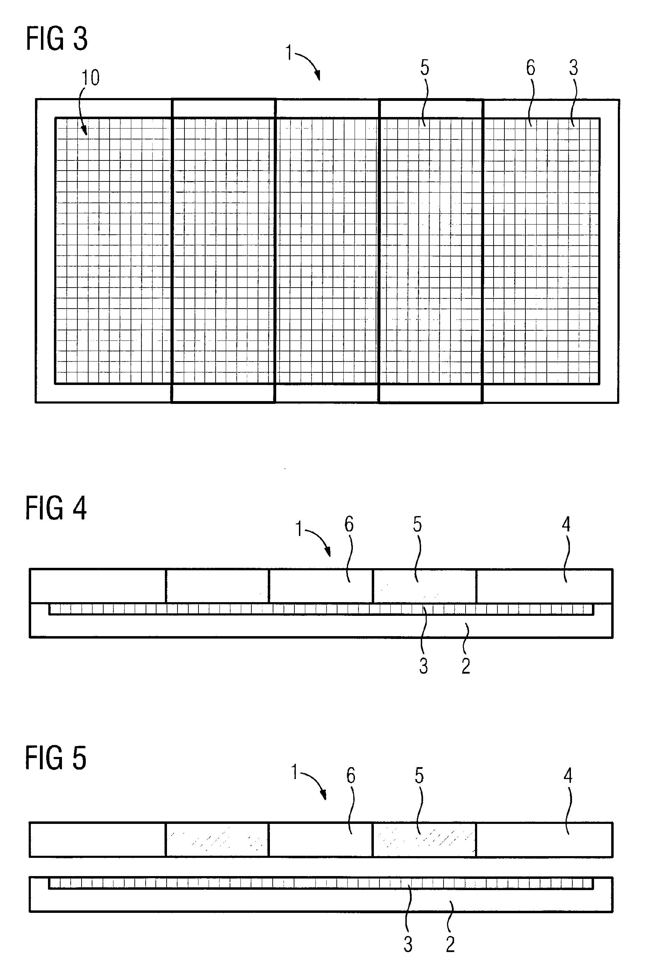

In Fig. 3 ist eine Vorrichtung mit einem ersten Träger 2 und einer porösen Schicht des zweiten Trägers 4 gezeigt, welche im Unterschied zur Vorrichtung in Fig. 2 funktionelle Bereiche in Balkenform bzw. Streifenform statt kreisrunder Form bzw. Zylinderform aufweist. Der Träger 4 in Fig. 3 weist eine Längsachse von der linken zur rechten Seite der Fig. 3 auf. Senkrecht zur Längsachse in der Zeichnungsebene weist der Träger 4 eine Querachse auf. Querachsen des Trägers 4 bzw. der porösen Schicht des Trägers 4 sind identisch zu Längsachsen der Balken-Form oder Streifen-Form der funktionellen Bereiche 5. Die in Fig. 3 dargestellten funktionellen Bereiche 5 sind sowohl entlang ihrer Längsachse als auch entlang ihrer Höhe vollständig durchgängig durch die poröse Schicht ausgebildet. Alternativ können sie auch nur teilweise entlang ihrer Längsachse und/oder entlang ihrer Höhe im bzw. auf dem porösen Material ausgebildet sein. Mit Höhe der funktionellen Bereiche 5 ist dabei die Ausdehnung bezeichnet entlang einer Achse, welche senkrecht zur Längsachse und Querachse des Trägers 4 ausgebildet ist.

In Fig. 4 ist ein Querschnitt entlang einer Längsachse der in Fig. 3 dargestellten Vorrichtung gezeigt. Die funktionellen Bereiche 5 und die nicht funktionalisierten Bereiche 6, welche in der porösen Schicht des zweiten Trägers 4 ausgebildet sind, stehen in direktem Kontakt zum Sensor-Array 3, welches auf der Vorderseite des ersten Trägers 2 ausgebildet ist. Die durchgehend durch die poröse Schicht 4 ausgebildeten funktionellen Bereiche 5 trennen den nicht funktionalisierten Bereich 6 in mehrere räumlich vollständig voneinander getrennte und beabstandete nicht funktionalisierte Bereich 6. Funktionelle und nicht funktionalisierte Bereiche 5 bzw. 6 sind alternierend in der porösen Schicht 4 entlang ihrer Längsachse angeordnet. Die balkenförmigen funktionellen Bereiche 5 ergeben Quader, in deren Volumen die Fänger-Moleküle 8 (vgl. Fig. 6) gleichmäßig verteilt immobilisiert sind. In unterschiedlichen funktionellen Bereichen 5 können unterschiedliche Fänger-Moleküle 8 angeordnet sein, womit unterschiedliche funktionelle Bereiche 5 zum Nachweis von unterschiedlichen Substanzen 9 aus der Flüssigkeit mit nachzuweisenden Substanzen dienen.

In Fig. 5 ist die Vorrichtung der Fig. 4 dargestellt, im Zustand bevor oder nachdem der erste Träger 2 mit dem zweiten Träger 4 in Kontakt gebracht wurde. Der erste Träger 2 kann wiederverwertet werden, wobei der zweite Träger als Einweg-Träger ausgewechselt wird. So können z. B. bei der Untersuchung von Patientenproben unterschiedlicher Patienten Kosten gespart werden. Zwischen zwei Verwendungen des Trägers 2 kann dieser gereinigt oder desinfiziert werden.

In der Fig. 6 ist die in Fig. 4 dargestellte Vorrichtung im Betrieb schematisch gezeigt. Ein Tropfen mit zu untersuchender Flüssigkeit mit nachzuweisenden Substanzen 9 wird auf die poröse Schicht 4 gegeben. Auf Grund von Kapillarkräften wird die zu untersuchende Flüssigkeit von der porösen Schicht 4 aufgesogen, so dass die poröse Schicht 4 weitgehend oder vollständig mit zu untersuchender Flüssigkeit befüllt bzw. durchtränkt ist. In funktionellen Bereichen 5 binden die nachzuweisenden Substanzen 9 an die immobilisierten Fänger-Moleküle 8 spezifisch.

Darauffolgend wird die poröse Schicht 4 gespült mit z. B. Reinst-Wasser oder Puffer-Lösung. Nichtgebundene Substanzen und zu untersuchende Flüssigkeit werden so aus der porösen Schicht 4 entfernt. Die gebundenen nachzuweisenden Substanzen 9 können direkt elektrochemisch nachgewiesen werden, wie es in Fig. 6 schematisch angedeutet ist. Alternativ können in einer Flüssigkeit gelöste Marker-Moleküle in die poröse Schicht 4 gegeben werden, welche an die spezifisch gebundenen nachzuweisenden Substanzen spezifisch, d.h. nur an nachzuweisende Substanzen binden. Die Marker-Moleküle werden dann elektrochemisch direkt oder indirekt z. B. über ein Redoxcycling nachgewiesen.

Die Marker-Moleküle können mit Labeln versehen sein, z. B. mit an die Marker-Moleküle gebundenen Enzym-Labeln. Bei einem elektrochemischen Nachweis werden z. B. an den Enzym-Labeln Substrat-Moleküle umgesetzt, insbesondere von einem Edukt zu einem Produkt. An der aktiven Sensor-Oberfläche bzw. der Elektrode werden die umgesetzten Substrat-Moleküle (Produkte) oxidiert bzw. reduziert, wobei ein Ladungstransfer über die Elektrode gemessen werden kann oder eine Änderung der Ladung nahe der Elektrode (insbesondere ein Strom oder eine Änderung der Spannung). Wird ein Sensor aus wenigstens zwei Elektroden entgegen gesetzter Polung (positiv + / negativ -) aufgebaut, so kann an der einen Elektrode (+) das Produkt oxidiert werden zu einem oxidiertem Produkt (Ox), und an der anderen Elektrode (-) das oxidierte Produkt wieder reduziert werden zu (Red).

Die an den Elektroden umgesetzte Ladungsmenge kann zeitabhängig gemessen werden und ist ein Maß für den Umsatz von Edukt zu Produkt am Enzym-Label und somit für das spezifische Binden von nachzuweisender Substanz 9 an Fänger-Moleküle 8. Nur bei Vorhandensein des für das Fänger-Molekül spezifischen nachzuweisenden Substanz-Moleküls 9 in der zu analysierenden Flüssigkeit erfolgt an dem zugeordneten Sensor ein zeitabhängiger Ladungsumsatz, welcher gemessen wird.

Wie in Fig. 6 als Vergrößerung dargestellt ist, besteht oder umfasst der Sensor 10 zum Nachweis der gebundenen nachzuweisenden Substanzen 9 elektrochemische Elektroden, welche z. B. als Interdigital-Elektroden ausgebildet sein können. Die Interdigital-Elektroden können kammartig ausgebildet sein, mit einem Abstand von z. B. einem Mikrometer zwischen benachbarten Stegen zweier Elektroden und einer Breite der Stege von jeweils z. B. einem Mikrometer. Ein Sensor 10 kann aus zwei ineinander greifenden kammartigen Interdigitalelektroden bestehen, wobei der Sensor 10 einen Durchmesser von z. B. 150 Mikrometer aufweist.

Fingerförmige Goldelektroden sind in Flüssigkeiten chemisch besonders innert und gut geeignet als Arbeitselektroden für elektrochemische Strom- und/oder Spannungs-Messungen. Als Messmethoden kommen unter anderem Amperometrie, Voltammetrie, Coulometrie oder Impedanz-Spektroskopie zur Anwendung. Als Referenz- und Gegenelektroden können ebenfalls Goldelektroden oder Elektroden aus Materialien wie z. B. Platin, Silber oder Silber/Silber-Chlorid eingesetzt werden.

Die Elektroden der Sensoren 10 sind photolithographisch leicht mit Standard-Prozessen der Halbleitertechnik herzustellen. Als Material für den ersten Träger 2 ist Silizium gut geeignet, aber auch andere Materialien wie z. B. Germanium, Gallium-Arsenid und andere Halbleiter sowie Isolator-Materialien wie Plastik, Epoxidharz oder Printed Circuit Boards sind geeignet. Der erste Träger 2 kann wie in Fig. 2 dargestellt als Chip ausgebildet sein, wobei unterhalb der Elektroden oder in einem gesonderten Bereich des Chips elektronische Schaltungen zur Verarbeitung der gemessenen Strom-Spannungs-Signale integriert werden können. In der Halbleitertechnik übliche Verfahren bei der Herstellung des ersten Trägers 2 mit den Sensoren 10, Isolierungen (z. B. Siliziumdioxid oder Photolack), integrierten Schaltungen und Kontakten ermöglichen die Herstellung des ersten Trägers 2 mit geringen Kosten.

Als zweiter Träger 4 können handelsübliche Teststreifen, wie sie z. B. für Schwangerschafts-Test, Glukose-Tests und Urin sowie Blut-Tests erhältlich sind, dienen. Die poröse Schicht des zweiten Trägers 4 kann unter anderem aus Nitrozellulose, Papier oder Gewebe hergestellt sein oder diese Materialien enthalten.

Die Vorrichtung kann in einer Durchflusszelle verwendet werden oder in einem Gehäuse integriert sein. Besonders kostengünstige Gehäuse sind aus Plastik herzustellen. Kühl- und/ oder Heizvorrichtungen können vorgesehen sein, um Reaktionen über die Temperatur zu steuern.

## Patentansprüche

1. Vorrichtung zum Nachweis von chemischen oder biochemischen Substanzen in Flüssigkeiten (1) mit einem ersten und einem zweiten Träger (2, 4),
wobei eine Oberfläche des ersten Trägers (2) ein Sensor-Array mit mehreren elektrochemischen Sensoren (3) umfasst, und wobei der zweite Träger eine poröse Schicht (4) aufweist mit wenigstens einem funktionellen Bereich (5), in dem spezifisch bindende Fänger-Moleküle (8) immobilisiert sind, wobei der wenigstens eine funktionelle Bereich (5) direkt benachbart zu wenigstens einem nicht funktionalisierten Bereich (6) angeordnet ist und wobei die Oberfläche des ersten Trägers (2) und die poröse Schicht des zweiten Trägers (4) direkt oder indirekt über ein flüssiges Medium miteinander in Kontakt stehen,
**dadurch gekennzeichnet, dass** dem wenigstens einen funktionellen Bereich (5) und dem wenigstens einen nicht funktionalisierten Bereich (6) jeweils mehrere Sensoren (10) des Sensor-Arrays (3) zugeordnet sind.

2. Vorrichtung (1) nach Anspruch 1, **dadurch gekennzeichnet, dass** die poröse Schicht (4) wenigstens zwei unterschiedliche, voneinander räumlich beabstandete funktionelle Bereiche (5) aufweist, wobei sich insbesondere mindestens ein Bereich von mindestens einem weiteren Bereich hinsichtlich seiner Fänger-Moleküle (8) unterscheidet.

3. Vorrichtung (1) nach Anspruch 2, **dadurch gekennzeichnet, dass** jeder Sensor (10) des Sensor-Arrays (3) eine aktive Fläche aufweist und die aktiven Flächen der Sensoren in einer ersten Ebene angeordnet sind, und Oberflächen der wenigstens zwei funktionellen Bereiche (5) eine zweite Ebene ausbilden, welche der ersten Ebene gegenüberliegend angeordnet ist, wobei jeweils die Oberfläche eines funktionellen Bereichs (5) in der zweiten Ebene größer ist als die Summe der aktiven, in der ersten Ebene vorhandenen Flächen von Sensoren (10), die jeweils dem funktionellen Bereich (5) zugeordnet sind.

4. Vorrichtung (1) nach einem der vorhergehenden Ansprüche, **dadurch gekennzeichnet, dass** die Fänger-Moleküle (8) räumlich dreidimensional verteilt im porösen Material immobilisiert sind.

5. Vorrichtung (1) nach einem der vorhergehenden Ansprüche, **dadurch gekennzeichnet, dass** die Sensoren (10) rechteckigen, insbesondere quadratischen Umfang aufweisen und/oder in Form eines Pixel-Arrays regelmäßig auf der Oberfläche des ersten Trägers (2) angeordnet sind.

6. Vorrichtung (1) nach einem der vorhergehenden Ansprüche, **dadurch gekennzeichnet, dass** beabstandete funktionelle Bereiche (5) in Form eines Arrays in und/oder auf der porösen Schicht (4) angeordnet sind, mit einer Anzahl m von unterschiedlichen funktionellen Bereichen (5) mit im wesentlichen gleichen räumlichen Abstand jeweils voneinander.

7. Vorrichtung (1) nach Anspruch 6, **dadurch gekennzeichnet, dass** das Sensor-Array (3) eine Anzahl von n Sensoren (10) aufweist, welche funktionellen Bereichen (5) zugeordnet sind, und die Anzahl n der zugeordneten Sensoren (10) zu funktionellen Bereichen (5) das x-fache der Anzahl m der funktionellen Bereiche (5) beträgt, mit x größer oder gleich zwei.

8. Vorrichtung (1) nach Anspruch 7, **dadurch gekennzeichnet, dass** die Anzahl m der funktionellen Bereiche (5) 8 beträgt und die Anzahl n der Sensoren (10) 32, und jedem funktionellen Bereich (5) 4 Sensoren (10) zugeordnet sind, oder dass die Anzahl m der funktionellen Bereiche (5) 24 beträgt und die Anzahl n der Sensoren (10) 384, und jedem funktionellen Bereich (5) 16 Sensoren (10) zugeordnet sind, oder die Anzahl m der funktionellen Bereiche (5) zwischen 8 und 24 beträgt und die Anzahl n der Sensoren (10) zwischen 32 und 384 beträgt.

9. Vorrichtung (1) nach einem der Ansprüche 1 bis 5, **dadurch gekennzeichnet, dass** die funktionellen Bereiche (5) in Form von Streifen oder Balken ausgebildet sind, insbesondere quaderförmigen Balken, die begrenzt werden von zwei im wesentlichen parallelen, voneinander beabstandeten Schnittflächen entlang von Querschnittsachsen der porösen Schicht (4) durch die poröse Schicht (4).

10. Vorrichtung (1) nach einem der vorhergehenden Ansprüche, **dadurch gekennzeichnet, dass** die Sensoren (10) aus Interdigital-Elektroden aufgebaut sind und/oder aus unbeschichteten Goldelektroden aufgebaut sind.

11. Vorrichtung (1) nach einem der vorhergehenden Ansprüche, **dadurch gekennzeichnet, dass** die poröse Schicht des zweiten Trägers (4) eine Membran umfasst, insbesondere aus Zellulose, Nitro-Zellulose, Lateral-Flow-Papier oder einem Gewebe.

12. Vorrichtung (1) nach einem der vorhergehenden Ansprüche, **dadurch gekennzeichnet, dass** die poröse Schicht (4) die Form eines Blattes aufweist, insbesondere die Form eines Papier-Streifens.

13. Vorrichtung (1) nach einem der vorhergehenden Ansprüche, **dadurch gekennzeichnet, dass** die Fänger-Moleküle (8) Antikörper oder Antigene, oder Aptamere, oder DNA-Fragmente, oder RNA-Fragmente, oder Peptide umfassen.

14. Verfahren zum Herstellen einer Vorrichtung (1) nach einem der vorhergehenden Ansprüche, **dadurch gekennzeichnet, dass** auf einen ersten Träger (2), mit einem Sensor-Array (3) aufgebaut aus mehreren elektrochemischen Sensoren (10), ein zweiter Träger aufgebracht wird, wobei der zweite Träger wenigstens einen funktionellen Bereich (5) in oder auf einer porösen Schicht (4) umfasst, in dem spezifisch bindende Fänger-Moleküle (8) immobilisiert sind, und wobei der wenigstens eine funktionelle Bereich (5) direkt benachbart zu wenigstens einem nicht funktionalisierten Bereich (6) in oder auf der porösen Schicht (4) angeordnet wird, und wobei durch das Aufbringen dem wenigstens einen funktionellen Bereich (5) und dem wenigstens einen nicht funktionalisierten Bereich (6) jeweils mehrere Sensoren (10) des Sensor-Arrays (3) zugeordnet werden.

15. Verfahren nach Anspruch 14, **dadurch gekennzeichnet, dass** der wenigstens eine funktionelle Bereich (5) in einem ersten Schritt in die poröse Schicht (4) durch Dispensieren, Drucken und/oder Spotten eingebracht wird, und in einem zweiten, zeitlich darauffolgenden Schritt die poröse Schicht (4) in direkte oder indirekte Verbindung gebracht wird mit dem Sensor-Array (3) des ersten Trägers (2), insbesondere durch Auflegen des zweiten Trägers auf den ersten Träger (2), und bei Einbringen von Flüssigkeit in die poröse Schicht (4) der wenigstens eine funktionelle Bereich (5) und der wenigstens eine nicht funktionalisierte Bereich (6) jeweils mit den mehreren zugeordneten Sensoren (10) über die Flüssigkeit miteinander in Verbindung treten.

16. Verwendung einer Vorrichtung (1) nach einem der Ansprüche 1 bis 13, **dadurch gekennzeichnet, dass** eine Flüssigkeit mit nachzuweisenden Substanzen (9) auf einen zweiten Träger (2) beaufschlagt wird, welcher eine poröse Schicht (4) mit wenigstens einem funktionellen Bereich (5) umfasst, in dem spezifisch bindende Fänger-Moleküle (8) zum Binden an die nachzuweisenden Substanzen (9) immobilisiert sind, wobei der wenigstens eine funktionelle Bereich (5) direkt benachbart zu wenigstens einem nicht funktionalisierten Bereich (6) in der porösen Schicht (4) angeordnet ist, und die Flüssigkeit insbesondere durch Kapillarkräfte zumindest teilweise durch die poröse Schicht (4) hindurchtransportiert wird, wobei die nachzuweisenden Substanzen (9) spezifisch an die Fänger-Moleküle (8) binden, und mit Hilfe von dem wenigstens einem funktionellen Bereich (5) zugeordneten Sensoren (10) eines Sensor-Arrays (3) auf einem zweiten Träger die gebundenen Substanzen (9) direkt oder indirekt elektrochemisch nachgewiesen werden, und wobei Sensoren (10) des Sensor-Arrays (3) auf dem zweiten Träger, welche dem wenigstens einem nicht funktionalisierten Bereich (6) zugeordnet sind, elektrochemisch nachweisen, dass keine nachzuweisenden Substanzen (9) in dem nicht funktionalisierten Bereich (6) gebunden wurden.

## Claims

1. Device for detecting chemical or biochemical substances in liquids (1) comprising a first and a second carrier (2, 4), wherein a surface of the first carrier (2) comprises a sensor array having a plurality of electrochemical sensors (3), and wherein the second carrier has a porous layer (4) having at least one functional region (5) in which specifically binding catcher molecules (8) are immobilized, wherein the at least one functional region (5) is arranged directly adjacent to at least one non-functionalized region (6), and wherein the surface of the first carrier (2) and the porous layer of the second carrier (4) are in contact with one another directly or indirectly via a liquid medium,
**characterized in that** in each case a plurality of sensors (10) of the sensor array (3) are assigned to the at least one functional region (5) and the at least one non-functionalized region (6).

2. Device (1) according to Claim 1, **characterized in that** the porous layer (4) has at least two different functional regions (5) which are spatially at a distance from one another, wherein in particular at least one region differs from at least one further region with regard to its catcher molecules (8).

3. Device (1) according to Claim 2, **characterized in that** each sensor (10) of the sensor array (3) has an active area and the active areas of the sensors are arranged in a first plane, and surfaces of the at least two functional regions (5) form a second plane, which is arranged such that it lies opposite the first plane, wherein in each case the surface of a functional region (5) in the second plane is larger than the sum of the active areas - present in the first plane - of sensors (10) which are in each case assigned to the functional region (5).

4. Device (1) according to any of the preceding claims, **characterized in that** the catcher molecules (8) are immobilized in a manner spatially distributed three-dimensionally in the porous material.

5. Device (1) according to any of the preceding claims, **characterized in that** the sensors (10) have a rectangular, in particular square, circumference and/or are arranged in the form of a pixel array regularly on the surface of the first carrier (2).

6. Device (1) according to any of the preceding claims, **characterized in that** spaced-apart functional regions (5) are arranged in the form of an array in and/or on the porous layer (4), with a number m of different functional regions (5) at substantially the same spatial distance respectively from one another.

7. Device (1) according to Claim 6, **characterized in that** the sensor array (3) has a number of n sensors (10) assigned to functional regions (5), and the number n of the assigned sensors (10) with respect to functional regions (5) is x times the number m of the functional regions (5), where x is greater than or equal to two.

8. Device (1) according to Claim 7, **characterized in that** the number m of functional regions (5) is 8 and the number n of sensors (10) is 32, and 4 sensors (10) are assigned to each functional region (5), or **in that** the number m of functional regions (5) is 24 and the number n of sensors (10) is 384, and 16 sensors (10) are assigned to each functional region (5), or the number m of functional regions (5) is between 8 and 24 and the number n of sensors (10) is between 32 and 384.

9. Device (1) according to any of Claims 1 to 5, **characterized in that** the functional regions (5) are embodied in the form of strips or bars, in particular parallelepipedal bars, which are delimited by two substantially parallel sectional areas spaced apart from one another along cross-sectional axes of the porous layer (4) through the porous layer (4).

10. Device (1) according to any of the preceding claims, **characterized in that** the sensors (10) are constructed from interdigital electrodes and/or are constructed from uncoated gold electrodes.

11. Device (1) according to any of the preceding claims, **characterized in that** the porous layer of the second carrier (4) comprises a membrane, in particular composed of cellulose, nitrocellulose, lateral flow paper or a fabric.

12. Device (1) according to any of the preceding claims, **characterized in that** the porous layer (4) has the form of a sheet, in particular the form of a paper strip.

13. Device (1) according to any of the preceding claims, **characterized in that** the catcher molecules (8) comprise antibodies or antigens, or aptamers, or DNA fragments, or RNA fragments, or peptides.

14. Method for producing a device (1) according to any of the preceding claims, **characterized in that** a second carrier is applied to a first carrier (2), having a sensor array (3) constructed from a plurality of electrochemical sensors (10), wherein the second carrier comprises at least one functional region (5) in or on a porous layer (4), in which specifically binding catcher molecules (8) are immobilized, and wherein the at least one functional region (5) is arranged directly adjacent to at least one non-functionalized region (6) in or on the porous layer (4), and wherein, as a result of the application, in each case a plurality of sensors (10) of the sensor array (3) are assigned to the at least one functional region (5) and the at least one non-functionalized region (6).

15. Method according to Claim 14, **characterized in that** the at least one functional region (5) is introduced into the porous layer (4) by dispensing, printing and/or spotting in a first step, and, in a second step temporally succeeding the latter, the porous layer (4) is directly or indirectly linked with the sensor array (3) of the first carrier (2), in particular by the second carrier being placed onto the first carrier (2), and, upon liquid being introduced into the porous layer (4), the at least one functional region (5) and the at least one non-functionalized region (6) in each case with the plurality of assigned sensors (10) are linked with one another via the liquid.

16. Use of a device (1) according to any of Claims 1 to 13, **characterized in that** a liquid comprising substances (9) to be detected is applied to a second carrier (2), which comprises a porous layer (4) having at least one functional region (5) in which specifically binding catcher molecules (8) for binding to the substances (9) to be detected are immobilized, wherein the at least one functional region (5) is arranged directly adjacent to at least one non-functionalized region (6) in the porous layer (4), and the liquid is transported at least partly through the porous layer (4) in particular by means of capillary forces, wherein the substances (9) to be detected specifically bind to the catcher molecules (8), and the bound substances (9) are directly or indirectly electrochemically detected with the aid of sensors (10) of a sensory array (3) on a second carrier which are assigned to the at least one functional region (5), and wherein sensors (10) of the sensor array (3) on the second carrier which are assigned to the at least one non-functionalized region (6) detect electrochemically that no substances (9) to be detected were bound in the non-functionalized region (6).

## Revendications

1. Dispositif de détection de substances chimiques ou biochimiques dans des liquides ( 1 ) comprenant un premier et un deuxième supports ( 2,4 ),
dans lequel une surface du premier support ( 2 ) comprend une matrice de capteurs ayant plusieurs capteurs ( 3 ) électrochimiques et dans lequel le deuxième support a une couche ( 4 ) poreuse ayant au moins une région ( 5 ) fonctionnelle, dans laquelle des molécules ( 8 ) de fixation se fixant spécifiquement sont immobilisées, la au moins une région ( 5 ) fonctionnelle étant directement voisine d'au moins une région ( 6 ) non fonctionnalisée et dans lequel la surface du premier support ( 2 ) et la couche poreuse du deuxième support ( 4 ) sont en contact entre elles directement ou indirectement par l'intermédiaire d'un milieu fluide, **caractérisé en ce que** respectivement plusieurs capteurs ( 10 ) de la matrice ( 3 ) de capteurs sont associés à la au moins une région ( 5 ) fonctionnelle et à la au moins une région ( 6 ) non fonctionnalisée.

2. Dispositif ( 1 ) suivant la revendication 1,
**caractérisé en ce que** la couche ( 4 ) poreuse a au moins deux régions ( 5 ) fonctionnelles différentes à distance l'une de l'autre dans l'espace, notamment au moins une région se distinguant d'au moins une autre région en ce qui concerne ses molécules ( 8 ) de fixation.

3. Dispositif ( 1 ) suivant la revendication 2,
**caractérisé en ce que** chaque capteur ( 10 ) de la matrice ( 3 ) de capteurs a une face active, et les faces actives des capteurs sont disposées dans un premier plan et des surfaces des au moins deux régions ( 5 ) fonctionnelles forment un deuxième plan qui est disposé de manière opposé au premier plan, respectivement la surface d'une région ( 5 ) fonctionnelle dans le deuxième plan étant plus grande que la somme des surfaces actives, présentes dans le premier plan de capteurs ( 10 ), qui sont associés respectivement à la région ( 5 ) fonctionnelle.

4. Dispositif ( 1 ) suivant l'une des revendications précédentes,
**caractérisé en ce que** les molécules ( 8 ) de fixation sont immobilisées dans le matériau poreux en étant réparties dans l'espace suivant trois dimensions.

5. Dispositif ( 1 ) suivant l'une des revendications précédentes,
**caractérisé en ce que** les capteurs ( 10 ) ont un pourtour rectangulaire, notamment carré, et/ou sont disposés sous la forme d'une matrice de pixels régulièrement à la surface du premier support ( 2 ).

6. Dispositif ( 1 ) suivant l'une des revendications précédentes,
**caractérisé en ce que** des régions ( 5 ) fonctionnelles à distance sont disposées sous la forme d'une matrice dans/ou sur la couche ( 4 ) poreuse en ayant un nombre m de régions ( 5 ) fonctionnelles différentes à sensiblement le même intervalle dans l'espace les unes des autres.

7. Dispositif ( 1 ) suivant la revendication 6,
**caractérisé en ce que** la matrice ( 3 ) de capteurs a un nombre n de capteurs ( 10 ) qui sont associés à des régions ( 5 ) fonctionnelles et le nombre n des capteurs ( 10 ) associés aux régions ( 5 ) fonctionnelles représente x fois le nombre m des régions ( 5 ) fonctionnelles, x étant supérieur ou égal à 2.

8. Dispositif ( 1 ) suivant la revendication 7,
**caractérisé en ce que** le nombre m des régions ( 5 ) fonctionnelles est de 8 et le nombre n des capteurs ( 10 ) est de 32, et quatre capteurs ( 10 ) sont associés à chaque région ( 5 ) fonctionnelle ou **en ce que** le nombre m des régions ( 5 ) fonctionnelles est de 24, et le nombre n des capteurs ( 10 ) est de 384, et seize capteurs ( 10 ) sont associés à chaque région ( 5 ) fonctionnelles ou le nombre m des régions ( 5 ) fonctionnelles est compris en 8 et 24 et le nombre n des capteurs ( 10 ) est compris entre 32 et 384.

9. Dispositif ( 1 ) suivant l'une des revendications 1 à 5, **caractérisé en ce que** les régions ( 5 ) fonctionnelles sont sous la forme de bandes ou de barres, notamment de barres parallélépipédiques qui sont délimitées dans la couche ( 4 ) poreuse par deux faces de coupe sensiblement parallèles et à distance l'une de l'autre le long d'axes de section transversale de la couche ( 4 ) poreuse.

10. Dispositif ( 1 ) suivant l'une des revendications précédentes,
**caractérisé en ce que** les capteurs ( 10 ) sont constitués d'électrodes interdigitées et/ou d'électrodes en or non revêtues.

11. Dispositif ( 1 ) suivant l'une des revendications précédentes,
**caractérisé en ce que** la couche poreuse du deuxième support ( 4 ) comprend une membrane notamment en cellulose, en nitrocellulose, en papier lateral-flow ou en un tissu.

12. Dispositif ( 1 ) suivant l'une des revendications précédentes,
**caractérisé en ce que** la couche ( 4 ) poreuse a la forme d'une feuille, notamment la forme d'une bande de papier.

13. Dispositif ( 1 ) suivant l'une des revendications précédentes,
**caractérisé en ce que** les molécules ( 8 ) de fixation comprennent des anticorps ou des antigènes ou des aptamères ou des fragments d'ADN ou des fragments d'ARN ou des peptides.

14. Procédé de production d'un dispositif ( 1 ) suivant l'une des revendications précédentes,
**caractérisé en ce que** l'on dépose un deuxième support sur un premier support ( 2 ) ayant une matrice ( 3 ) de capteurs composée de plusieurs capteurs ( 10 ) électrochimiques, le deuxième support comprenant au moins une région ( 5 ) fonctionnelle dans ou sur une couche ( 4 ) poreuse dans laquelle des molécules ( 8 ) de fixation se fixant spécifiquement sont immobilisées et dans lequel on met la au moins une région ( 5 ) fonctionnelle au voisinage immédiat d'au moins une région ( 6 ) non fonctionnalisée dans ou sur la couche ( 4 ) poreuse et dans lequel, par le dépôt, on associe respectivement plusieurs capteurs ( 10 ) de la matrice ( 3 ) de capteurs à la au moins une région ( 5 ) fonctionnelle et à la au moins une région ( 6 ) non fonctionnalisée.

15. Procédé suivant la revendication 14,
**caractérisé en ce que** l'on met la au moins à une région ( 5 ) fonctionnelle, dans un premier stade, dans la couche ( 4 ) poreuse par distribution, par impression et/ou par formation de taches et, dans un deuxième stade venant ensuite dans le temps, on met la couche ( 4 ) poreuse en liaison directe ou indirecte avec la matrice ( 3 ) de capteurs du premier support
( 2 ), notamment en mettant le deuxième support sur le premier support ( 2 ) et en introduisant du liquide dans la couche ( 4 ) poreuse, on met la au moins une région ( 5 ) fonctionnelle et la au moins une région ( 6 ) non fonctionnalisée en liaison entre elles respectivement par les plusieurs capteurs ( 10 ) associés par l'intermédiaire du liquide.

16. Utilisation d'un dispositif ( 1 ) suivant l'une des revendications 1 à 13,
**caractérisé en ce que** l'on charge un liquide ayant des substances ( 9 ) à détecter sur un deuxième support ( 2 ) qui comprend une couche ( 4 ) poreuse ayant au moins une région ( 5 ) fonctionnelle dans laquelle des molécules ( 8 ) de fixation se fixant spécifiquement sont immobilisées pour la fixation sur les substances ( 9 ) à détecter, la au moins une région ( 5 ) fonctionnelle étant disposée directement au voisinage d'au moins une région ( 6 ) non fonctionnalisée de la couche ( 4 ) poreuse et le liquide traversant, notamment par des forces capillaires, au moins en partie la couche ( 4 ) poreuse, les substances ( 9 ) à détecter se fixant spécifiquement aux molécules ( 8 ) de fixation et, à l'aide de capteurs ( 10 ) associés à la au moins une région ( 5 ) fonctionnelle d'une matrice ( 3 ) de capteur sur un deuxième support, les substances ( 9 ) fixées étant détectées électrochimiquement directement ou indirectement et dans lequel des capteurs ( 10 ) de la matrice ( 3 ) de capteurs sur le deuxième support, qui sont associés à la au moins une région ( 6 ) non fonctionnalisée, détectent électrochimiquement que des substances à détecter ne sont pas fixées dans la région ( 6 ) non fonctionnalisée.
